(19) Europäisches Patentamt
European Patent Office
Office européen des brevets

(11) Veröffentlichungsnummer: **0 012 428**
**B1**

# (12) EUROPÄISCHE PATENTSCHRIFT

(45) Veröffentlichungstag der Patentschrift: **13.05.81**

(21) Anmeldenummer: **79105108.9**

(22) Anmeldetag: **12.12.79**

(51) Int. Cl.³: **C 07 C 125/08,** C 07 C 155/02, C 07 D 307/68, C 07 D 333/38, A 01 N 47/40

## (54) Substituierte Cyanamide, Verfahren zu ihrer Herstellung und ihre Verwendung als Fungizide.

(30) Priorität: **18.12.78 DE 2854600**

(43) Veröffentlichungstag der Anmeldung: **25.06.80 Patentblatt 80/13**

(45) Bekanntmachung des Hinweises auf die Patenterteilung: **13.05.81 Patentblatt 81/19**

(84) Benannte Vertragsstaaten: **AT BE CH DE FR GB IT LU NL SE**

(56) Entgegenhaltungen: **DE-A-1 643 527**

(73) Patentinhaber: **BASF Aktiengesellschaft, Carl-Bosch-Strasse 38, D-6700 Ludwigshafen (DE)**

(72) Erfinder: **Eicken, Karl, Dr. Dipl.-Chem., Waldstrasse 63, D-6706 Wachenheim (DE)**
Erfinder: **Plath, Peter, Dr. Dipl.-Chem., Berner Weg 24, D-6700 Ludwigshafen (DE)**
Erfinder: **Pommer, Ernst-Heinrich, Dr., Berliner Platz 7, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



ACTORUM AG.

# Substituierte Cyanamide, Verfahren zu ihrer Herstellung und ihre Verwendung als Fungizide

Die vorliegende Erfindung betrifft neue wertvolle substituierte Cyanamide, Verfahren zu ihrer Herstellung sowie Fungizide, die diese Verbindungen enthalten.

Es ist bereits bekannt, dass Cyanacetamid-Derivate z.B. das 2-Cyano-N-(äthylaminocarbonyl)-2-methoxy-iminoacetamid eine fungizide Wirkung gegenüber niederen Pilzen hat (US-PS 3954992). Es ist ferner bekannt, das N-Trichlormethylthio-tetrahydrophthalimid zur Bekämpfung von Pilzen zu verwenden (Chemical Week, June 21,1972, Seite 46).

Aus der DE-OS 1643527 sind Aminosäurederivate mit herbiziden Eigenschaften bekannt, die sich in ihrer chemischen Struktur wesentlich von den neuen Verbindungen unterscheiden und auch keinen Hinweis auf die fungizide Wirkung dieser Verbindungen geben können.

Es wurde gefunden, dass substituierte Cyanamide der Formel

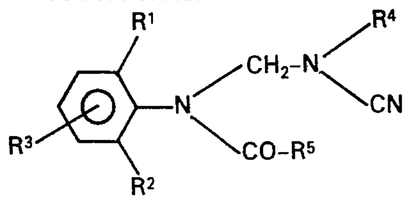

I

in welcher

$R^1$ $C_1$–$C_4$ Alkyl oder $C_1$–$C_4$-Alkoxy,

$R^2$ Wasserstoff, $C_1$–$C_4$ Alkyl, $C_1$–$C_4$ Alkoxy oder Halogen,

$R^3$ Wasserstoff, $C_1$–$C_4$-Alkyl oder Halogen,

$R^4$ $C_1$–$C_6$-Alkoxcarbonyl, $C_3$–$C_4$-Alkenoxycarbonyl, $C_1$–$C_6$-Alkyl-thiocarbonyl oder Cyan und

$R^5$ ein gegebenenfalls durch niederes Alkoxy, Alkylthio, Cyan oder Halogen substituiertes $C_1$–$C_6$ Alkyl, ein gegebenenfalls durch Halogen substituiertes $C_2$–$C_5$ Alkenyl, ein $C_2$–$C_4$ Alkinyl, ein $C_3$–$C_7$ Cycloalkyl, einen gegebenenfalls durch niederes Alkyl oder Halogen substituierten heterocyclischen Rest bedeutet, der ein Sauerstoffatom oder ein Schwefelatom oder ein Stickstoffatom oder bis zu zwei Stickstoffatome und ein Sauerstoffatom oder bis zu zwei Stickstoffatome und ein Schwefelatom enthält, $R^5$ ferner ein Phenyl oder ein durch ein bis drei gleiche oder verschiedene Substituenten aus der Gruppe Fluor, Chlor, Brom, Trifluormethyl, Cyan, Nitro, $C_1$–$C_4$-Alkyl, $C_1$–$C_4$ Alkoxy substituiertes Phenyl bedeutet,

eine gute fungizide Wirkung gegenüber phytopathogenen Pilzen haben.

Es wurde ferner gefunden, dass man substituierte Cyanamide erhält, wenn man ein N-Halogenmethylanilid der Formel

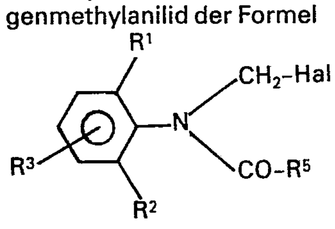

II

in welcher $R^1$, $R^2$, $R^3$ und $R^5$ die oben angegebene Bedeutung haben und Hal Halogen, insbesondere Chlor oder Brom bedeutet, mit einem Cyanamid der Formel

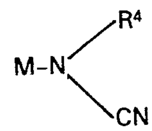

III

in welcher $R^4$ die oben angegebene Bedeutung hat und M Wasserstoff, ein Alkalimetall oder einen Tetraalkylammonium-Rest bedeutet, gegebenenfalls in Gegenwart eines Säurebindemittels und gegebenenfalls in Gegenwart eines gegenüber den Reaktionspartnern inerten Lösungsmittels umsetzt.

Unter Alkyl und Alkylrest einer Alkoxygruppe bei den Resten $R^1$, $R^2$, $R^3$, $R^4$ und $R^5$ sind je nach Zahl der genannten Kohlenstoffatome beispielsweise folgende Gruppen zu verstehen: Methyl, Äthyl, n-Propyl, iso-Propyl, n-, iso-, sec.- oder tert.-Butyl, Pentyl oder Hexyl und ihre Isomeren.

Als Alkenyl sind vor allem beispielsweise Vinyl, Allyl, Methallyl und Pentenyle zu nennen. Als $C_2$ bis $C_4$-Alinyl sind vor allem beispielsweise Äthinyl, Propargyl und But-2-inyl zu erwähnen.

Als Cycloalkyl ist vor allem beispielsweise Cyclopropyl, Cyclobutyl, Cyclopentyl und Cyclohexyl zu nennen.

Unter Halogen sind beispielsweise Fluor, Chlor oder Brom zu verstehen.

Als heterocyclischer Rest in der Bedeutung für $R^5$ sind vorzugsweise 5- bis 6gliedrige Reste zu nennen beispielsweise Furan, Thiophen, Oxazol, Isoxazol, Thiazol, Oxadiazol, Thiodiazol, Tetrahydrofuran, 2,3-Dihydropyran oder Pyridin, welche gegebenenfalls durch Methyl und/oder Halogen substituiert sein können.

Als substituiertes Phenyl für $R^5$ gelten vor allem beispielsweise mono-substituierte Phenyle wie Fluorphenyl, Chlorphenyl, Bromphenyl, Trifluormethylphenyl, Tolyl, Anisyl, Nitrophenyl; ferner disubstituierte Phenyle wie Dichlorphenyl, Difluorphenyl, Fluorchlorphenyl, Dimethylphenyl, Dimethoxyphenyl, Methylfluorphenyl, Methylchlorphenyl, Methylbromphenyl, Methoxychlorphenyl, Methyltrifluormethylphenyl, Chlortrifluormethylphenyl.

Die Umsetzungen können in Anwesenheit oder Abwesenheit von Lösungsmitteln durchgeführt werden, gegenüber denen die Reaktionspartner inert sind. Als Lösungsmittel kommen z.B. aromatische Kohlenwasserstoffe wie Toluol, Xylol; halogenierte Kohlenwasserstoffe wie Chlorbenzol, Chloroform, Methylenchlorid, Methylchlorid; Äther wie Tetrahydrofuran, Dioxan; Nitrile wie Acetonitril; N,N-Dialkylamide wie Dimethylformamid; Sulfone wie Dimethylsulfoxid; Ketone wie Aceton; aliphatische Ester wie Essigsäureester und Gemische dieser Lösungsmittel in Frage. Die Reaktionstemperaturen liegen zwischen

0° und 150°C, vorzugsweise zwischen 20° und 100°C.

Setzt man Cyanamide der Formel III um, in der M die Bedeutung von Wasserstoff hat, dann ist die Verwendung von säurebindenden Mitteln vorteilhaft. Als solche kommen tertiäre Amine z.B. Triäthylamin, Pyridin und substituierte Pyridine oder anorganische Basen, z.B. Oxide und Hydroxide, Carbonate und Hydrogencarbonate von Alkalimetallen in Betracht.

Es wurde ferner eine besondere Ausführungsform für die Herstellung der Cyanamid-Verbindungen der allgemeinen Formel I gefunden, wobei man ein N-Halogenmethylanilid der Formel

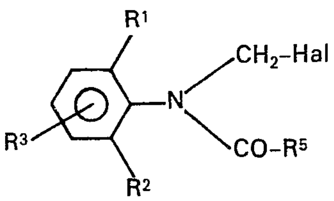

II

in welcher $R^1$, $R^2$, $R^3$ und $R^5$ die oben angegebene Bedeutung haben und Hal Chlor oder Brom bedeutet,

mit einem Cyanamid der Formel

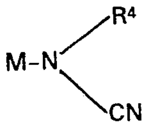

III

in welcher $R^4$ die oben angegebene Bedeutung hat und M ein Alkalimetall bedeutet, in einem Zweiphasensystem, wobei die eine Phase Wasser und die andere Phase ein mit der wässrigen Phase nicht mischbares gegenüber den Reaktionspartnern inertes Lösungsmittel ist, in Gegenwart eines Phasentransferkatalysators umsetzt. Gegebenenfalls kann die Umsetzung auch in einem Zweiphasensystem in Gegenwart eines Phasentransferkatalysators ohne Zusatz von Wasser durchgeführt werden, wobei das Cyanamid der Formel III, in dem mit Wasser nicht mischbaren Lösungsmittel suspendiert wird.

Das mit Wasser nicht mischbare Lösungsmittel ist vorzugsweise ein aromatischer Kohlenwasserstoff oder ein halogenierter Kohlenwasserstoff. Der Phasentransferkatalysator liegt beispielsweise in Mengen von 0,5 bis 30 Molprozent bezogen auf eingesetztes N-Halogenmethylanilid der Formel II vor.

Die Reaktion wird bei 0°C bis 100°C, vorzugsweise 10 bis 50°C vorgenommen.

Als Phasentransferkatalysatoren kommen Oniumverbindungen z.B. quaternäre Ammoniumverbindungen oder Phosphoniumverbindungen oder makrocyclische Polyäther in Frage. Quaternäre Ammoniumverbindungen sind z.B. Tetrabutylammoniumhydrogensulfat, Tetrapentylammoniumchlorid, Tetraoctylammoniumchlorid, Tripropylbutylammoniumchlorid, Tricaprylyl-methylammoniumchlorid, Hexadecyl-trimethylammoniumchlorid, Distearyl-dimethylammoniumchlorid, Dibenzyl-dimethylammonium-methylsulfat, Dimethyl-dodecyl-benzyl-ammoniumchlorid, Benzyl-trimethylammoniumchlorid, Benzyl-triäthylammoniumchlorid, 2-Hydroxy-äthyltrimethylammoniumchlorid, beispielsweise quaternäre Phosphoniumverbindungen, z.B. Tributyl-methylphosphoniumbromid, Hexadecyl-tributylphosphoniumbromid, Äthyl-triphenyl-phosphoniumbromid, Tetraphenylphosphoniumbromid oder die entsprechenden Hydroxide dieser Onium-Verbindungen.

Makrocyclische Polyäther sind z.B. 15-Krone-5, 18-Krone-6, Dibenzo-18-krone-6, Dicyclohexyl-18-krone-6 (Kronenäther).

Zur Herstellung der neuen Verbindungen setzt man auf 1 Mol N-Halogenmethylanilid der Formel II mindestens 1 Mol Cyanamid der Formel III ein.

Zur Isolierung der Verbindungen der Formel I wird beispielsweise, gegebenenfalls nach Abfiltrieren des entstandenen Halogenids, das Filtrat eingedampft und der Rückstand in einem organischen mit Wasser nicht mischbaren Lösungsmittel gelöst. Dann wird die organische Phase mit Wasser gewaschen, getrocknet und danach im Vakuum eingeengt. Das erhaltene Produkt der Formel I ist in vielen Fällen rein, wenn nötig kann es durch Umkristallisation oder Chromatographie beispielsweise an Kieselgel weiter gereinigt werden.

Bei dem zweiphasigen Herstellungsverfahren wird nach Ende der Umsetzung die organische Phase abgetrennt bzw. von Ungelöstem abfiltriert, mit Wasser gewaschen, getrocknet und wie oben beschrieben der Wirkstoff der Formel I isoliert.

Die als Ausgangsverbindungen verwendeten N-Halogenmethylanilide sind teilweise bekannt (US-PS 3637847) oder können in an sich bekannter Weise durch Umsetzung von entsprechend substituierten Phenylazomethinen mit Säurechloriden der Formel $R^5$–CO–Hal hergestellt werden, wobei $R^5$ die oben genannten Bedeutungen hat und Hal Halogen bedeutet.

Cyanamid-Verbindungen der Formel III sind bekannte Verbindungen, beispielsweise: Cyancarbaminsäureester (DE-PS 2474453, DE-OS 1795849) Cyancarbaminsäurethiolester (Yuki Cosei Kagaku Kyokai Shi 1971, 29 (1) 67 (CA: 74, 140877 u)) und Dicyanimid (Liebigs Ann. Chem. 427, 1 1922; J. Chem. Soc. C, 1970, 875).

Als Beispiele für die als Ausgangsprodukte geeigneten Cyanamid-Verbindungen sowie deren Salze seien im einzelnen genannt:

Cyancarbaminsäuremethylester oder dessen Natrium-, Kalium- oder Tetramethylammoniumsalze

Cyancarbaminsäureäthylester oder dessen Natrium-, Kalium- oder Tetramethylammoniumsalz,

Cyancarbaminsäure-n-propylester oder dessen Natrium-, Kalium- oder Tetramethylammoniumsalz,

Cyancarbaminsäure-isopropylester oder dessen Natrium- oder Kaliumsalz,

Cyancarbaminsäure-n-butylester oder dessen Natrium- oder Kaliumsalz,

Cyancarbaminsäure-sec.-butylester oder dessen Natrium- oder Kaliumsalz,
Cyancarbaminsäure-n-hexylester oder dessen Natrium- oder Kaliumsalz,
Cyancarbaminsäure-methylthiolester oder dessen Natrium-, Kalium- oder Tetramethylammoniumsalz,
Cyancarbaminsäure-äthylthiolester oder dessen Natrium-, Kalium- oder Tetraäthylammoniumsalz,
Cyancarbaminsäure-n-propylthiolester oder dessen Natrium- oder Kaliumsalz,
Cyancarbaminsäure-isopropylthiolester oder dessen Natrium- oder Kaliumsalz.
Dicyanimid, insbesondere dessen Natrium-, Kalium- oder Tetramethylammoniumsalz.

Die folgenden Beispiele erläutern die Herstellung der neuen Cyanamid-Verbindungen der Formel I sowie die Herstellung der Ausgangsmaterialien der Formel II.

In den Beispielen verhalten sich Gewichtsteile zu Volumenteilen wie Kilogramm zu Liter.

Beispiel a

315 Raumteile einer Toluol-Lösung, die 1 Mol 2,6-Dimethylphenylazomethin enthält, (US-PS 3637847) wurden unter Rühren bei 5-10°C zu einer Lösung von 130,5 Gewichtsteilen 2-Furancarbonsäurechlorid in 100 Raumteilen Toluol unter Kühlung zugetropft und anschliessend 10 Std. bei Raumtemperatur gerührt. Nach dem Kühlen, Absaugen und Trocknen im Vakuum erhielt man 198 Gewichtsteile N-Chlormethylfuran-2-carbonsäure-2',6'-dimethylanilid vom Schmelzpunkt 124 bis 126°C.

In analoger Weise können folgende N-Halogenmethylanilide der Formel II hergestellt werden:

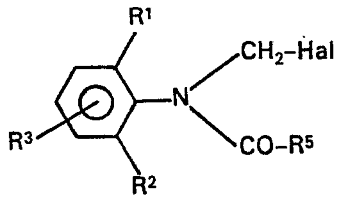

| $R^1$ | $R^2$ | $R^3$ | Hal | $R^5$ | Fp. °C |
|---|---|---|---|---|---|
| $CH_3$ | $CH_3$ | H | Cl | $CH_3$ | feste Masse |
| $CH_3$ | $C_2H_5$ | H | Cl | $CH_3$ | feste Masse |
| $C_2H_5$ | $C_2H_5$ | H | Cl | $CH_3$ | Öl |
| $CH_3$ | $CH_3$ | H | Cl | $C_2H_5$ | Öl |
| $CH_3$ | $C_2H_5$ | H | Cl | $C_2H_5$ | Öl |
| $C_2H_5$ | $C_2H_5$ | H | Cl | $C_2H_5$ | Öl |
| $CH_3$ | $CH_3$ | H | Cl | $CH(CH_3)_2$ | |
| $CH_3$ | $C_2H_5$ | H | Cl | $CH(CH_3)_2$ | |
| $C_2H_5$ | $C_2H_5$ | H | Cl | $CH(CH_3)_2$ | 75-76 |
| $C_2H_5$ | $C_2H_5$ | H | Cl | $C(CH_3)_3$ | Öl |
| $CH_3$ | $CH_3$ | H | Cl | $CH(Cl)CH_3$ | 95° |
| $C_2H_5$ | $CH_3$ | H | Cl | $CH(Cl)CH_3$ | |
| $C_2H_5$ | $C_2H_5$ | H | Cl | $CH(Cl)CH_3$ | |
| $CH_3$ | $CH_3$ | 2-$CH_3$ | Cl | $CH(Cl)CH_3$ | |
| $CH_3$ | $C_2H_5$ | H | Cl | $CH_2$-$CH_2Cl$ | zähes Öl |
| $CH_3$ | $CH_3$ | H | Cl | $CH_2$-$CH_2Cl$ | |
| $CH_3$ | $CH_3$ | H | Cl | $CH_2$-$CH_2$-<br>$CH_2Cl$ | |
| $CH_3$ | $CH_3$ | H | Cl | $CHCl_2$ | 102-104 |
| $CH_3$ | $CH_3$ | $CH_3$ | Cl | $CHCl_2$ | |
| $CH_3$ | $C_2H_5$ | H | Cl | $CHCl_2$ | 88-90 |
| $CH_3$ | $CH_3$ | H | Cl | $CCl_3$ | Öl |
| $CH_3$ | $CH_3$ | H | Cl | $CH=CH_2$ | |
| $CH_3$ | $CH_3$ | H | Cl | $C\equiv CH$ | |
| $CH_3$ | $CH_3$ | H | Cl | $C=CH_2$<br>$CH_3$ | 91-92 |
| $CH_3$ | $CH_3$ | H | Cl | $C=CH_2$<br>Cl | |
| $CH_3$ | $CH_3$ | H | Cl | $CH=CCl_2$ | |
| $CH_3$ | $CH_3$ | H | Cl | $CCl=CCl_2$ | |
| $CH_3$ | $CH_3$ | H | Cl | 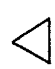 | 82 |
| $CH_3$ | $C_2H_5$ | H | Cl | 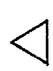 | Öl |

| $R^1$ | $R^2$ | $R^3$ | Hal | $R^5$ | Fp. °C |
|---|---|---|---|---|---|
| $C_2H_5$ | $C_2H_5$ | H | Cl | 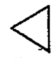 | Öl |
| $CH_3$ | $CH_3$ | H | Cl | 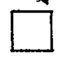 | |
| $CH_3$ | $CH_3$ | H | Cl | 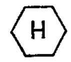 | |
| $CH_3$ | $C_2H_5$ | H | Cl | 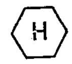 | |
| $C_2H_5$ | $C_2H_5$ | H | Cl | 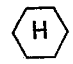 | 97–100 |
| $CH_3$ | $CH_3$ | H | Cl | $CH_2OCH_3$ | 70° |
| $CH_3$ | $CH_3$ | $CH_3$ | Cl | $CH_2OCH_3$ | |
| $CH_3$ | $C_2H_5$ | H | Cl | $CH_2OCH_3$ | |
| $C_2H_5$ | $C_2H_5$ | H | Cl | $CH_2OCH_3$ | Öl |
| $CH_3$ | $CH_3$ | H | Cl | $CH_2SCH_3$ | Kristallmasse |
| $CH_3$ | $C_2H_5$ | H | Cl | $CH_2SCH_3$ | 127 |
| $C_2H_5$ | $C_2H_5$ | H | Cl | $CH_2SCH_3$ | Öl |
| $CH_3$ | $CH_3$ | H | Cl | $CH_2-CN$ | |
| $CH_3$ | $CH_3$ | H | Cl | 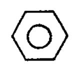 | 133 |
| $CH_3$ | $C_2H_5$ | H | Cl | 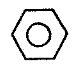 | |
| $C_2H_5$ | $C_2H_5$ | H | Cl | 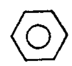 | 98–99 |
| $CH_3$ | $CH_3$ | H | Cl | 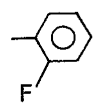 | 86–88 |
| $CH_3$ | $CH_3$ | H | Cl | 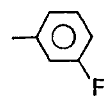 | 114–116 |
| $CH_3$ | $CH_3$ | H | Cl | 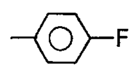 | 141–143 |
| $CH_3$ | $CH_3$ | H | Cl | 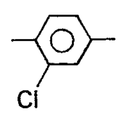 | 93 |
| $CH_3$ | $CH_3$ | H | Cl | 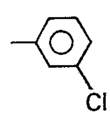 | 145 |
| $CH_3$ | $CH_3$ | H | Cl | 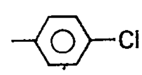 | 120 |
| $CH_3$ | $CH_3$ | H | Cl | 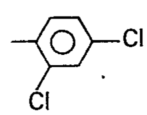 | 108 |
| $C_2H_5$ | $C_2H_5$ | H | Cl | 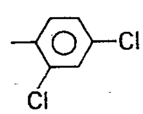 | |
| $CH_3$ | $CH_3$ | H | Cl | 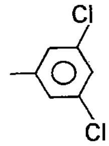 | 132 |

| $R^1$ | $R^2$ | $R^3$ | Hal | $R^5$ | Fp. °C |
|---|---|---|---|---|---|
| $CH_3$ | $CH_3$ | H | Cl | 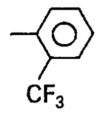 | |
| $C_2H_5$ | $C_2H_5$ | H | Cl | 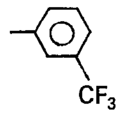 | 63–64 |
| $CH_3$ | $CH_3$ | H | Cl | 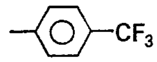 | 108–109 |
| $C_2H_5$ | $C_2H_5$ | H | Cl | 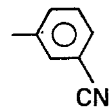 | 112–114 |
| $CH_3$ | $CH_3$ | H | Cl | 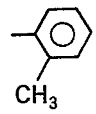 | 118–119 |
| $CH_3$ | $CH_3$ | H | Cl | 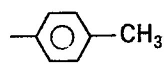 | 119–120 |
| $CH_3$ | $CH_3$ | H | Cl | 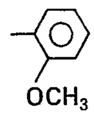 | |
| $CH_3$ | $CH_3$ | H | Cl | 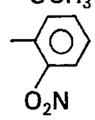 | 205–207 |
| $CH_3$ | $CH_3$ | H | Cl | 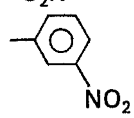 | 152–154 |
| $CH_3$ | $CH_3$ | H | Cl | 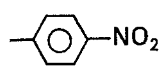 | |
| $CH_3$ | $CH_3$ | H | Cl | 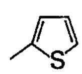 | 94–97 |
| $CH_3$ | $CH_3$ | H | Cl | 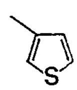 | 120–121 |
| $CH_3$ | $CH_3$ | H | Cl | 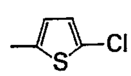 | |
| $CH_3$ | $CH_3$ | H | Cl | 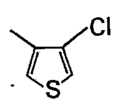 | 118 |
| $CH_3$ | $CH_3$ | H | Cl | 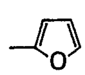 | 124–126 |
| $CH_3$ | $CH_3$ | H | Cl | 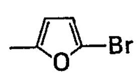 | |
| $C_2H_5$ | $C_2H_5$ | H | Cl | 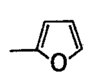 | 69–70 |

| $R^1$ | $R^2$ | $R^3$ | Hal | $R^5$ | Fp. °C |
|---|---|---|---|---|---|
| $CH_3$ | $CH_3$ | H | Cl | 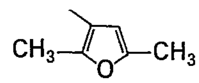 | 68–70 |
| $C_2H_5$ | $C_2H_5$ | H | Cl | 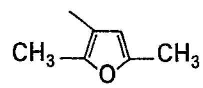 | |
| $CH_3$ | $CH_3$ | H | Cl | 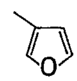 | |
| $CH_3$ | $CH_3$ | H | Cl | 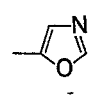 | |
| $CH_3$ | $CH_3$ | H | Cl | 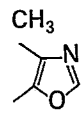 | 83–85 |
| $CH_3$ | $CH_3$ | H | Cl | 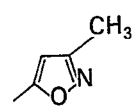 | 106 |
| $CH_3$ | $CH_3$ | H | | 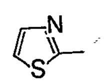 | |
| $CH_3$ | $CH_3$ | H | Cl | 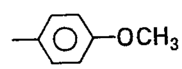 | 92–93 |
| $CH_3$ | $CH_3$ | H | Cl | 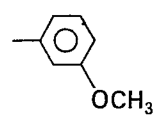 | 107–08 |
| $CH_3$ | $CH_3$ | H | Cl | 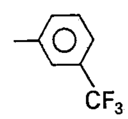 | 105 |
| $CH_3$ | $CH_3$ | H | Cl | 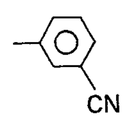 | 118–20 |
| $C_2H_5$ | $C_2H_5$ | H | Cl | 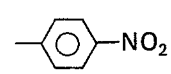 | 118–20 |
| $CH_3$ | H | 4–Cl | Cl | $CH_2OCH_3$ | Öl |
| $CH_3$ | $CH_3$ | H | Cl | $CH_2OC_2H_5$ | 80–81 |
| $CH_3$ | $CH_3$ | H | Cl | $CH_2$-$CH_2OCH_3$ | Öl |
| $CH_3$ | $CH_3$ | H | Cl | $CH(CH_3)OCH_3$ | Öl |

Beispiel 1

Eine Mischung aus 29,2 Gewichtsteilen N-Chlormethyl-furan-2-carbonsäure-2′, 6′-diäthylanilid und 13,4 Gewichtsteilen des Natriumsalzes des Cyancarbaminsäuremethylesters in 120 Volumenteilen Acetonitril wird bei 25 °C 12 Std. gerührt. Nach Verdampfen des Lösungsmittels im Vakuum wird der Rückstand in Essigester und Wasser gelöst. Die organische Phase ergibt nach dem Trocknen, Verdampfen des Lösungsmittels und Anreiben des Rückstandes mit Diisopropyläther 25,5 Teile N-Methoxycarbonyl-N-(N′-furyl-2-carbonyl, N′-2′, 6′-diäthylanilinomethyl)-cyanamid vom Fp.: 101–103 °C.

Beispiel 2

Zu einer Lösung von 12,2 Gewichtsteilen des Natriumsalzes des Cyancarbaminsäuremethylesters und 1 Teil Benzyl-triäthylammoniumchlorid in 30 Volumenteilen Wasser tropft man bei 20 bis 25°C eine Lösung von 26,4 Gewichtsteilen N-(Chlormethyl)-furan-2-carbonsäure-2′, 6′-dimethylanilid in 70 Volumenteilen Methylenchlorid unter intensiver Vermischung der Phasen. Nach 6stündiger Durchmischung wird die organische Phase abgetrennt, dreimal mit Wasser gewaschen und über Natriumsulfat getrocknet. Nach dem Verdampfen des Lösungsmittels isoliert man 29,0 Gewichtsteile N-Methoxycarbonyl-N-(N′-furyl-2-carbonyl, N′-2′, 6′-dimethylanilinomethyl)-cyanamid, die nach dem Umkristallisieren aus wenig Methanol einen Fp 96–98°C aufweisen.

Beispiel 3

24,1 Gewichtsteile N-Chlormethyl-benzoesäure-2,6-dimethylanilid werden in 100 Volumenteilen Acetonitril gelöst, 11,8 Gewichtsteile Natriumsalz des Cyancarbaminsäuremethylesters zugesetzt und die Mischung 24 Std. bei 25°C gerührt. Nach dem Abfiltrieren von Ungelöstem wird im Vakuum eingeengt und Rückstand mit Methylenchlorid und Wasser gelöst. Aus der organischen Phase isoliert man nach Verdampfen des Lösungsmittels und Umkristallisieren aus Methanol (100 ml) 18,0 Gewichtsteile N-Methoxycarbonyl-N-(N′-benzoyl, N′-2′,6′-dimethylanilinomethyl)-cyanamid vom Fp. 136°C.

Folgende Verbindungen erhält man in entsprechender Weise:

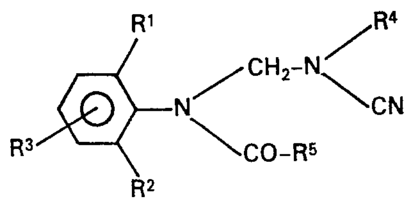

| Wirkstoff-Nr. | $R^1$ | $R^2$ | $R^3$ | $R^4$ | $R^5$ | Fp. (°C) oder $n_D^{20}$ |
|---|---|---|---|---|---|---|
| 1 | $CH_3$ | $CH_3$ | H | $CO_2CH_3$ | $CH_3$ | 112–114 |
| 2 | $CH_3$ | $CH_3$ | H | $CO_2C_2H_5$ | $CH_3$ | 78–80 |
| 3 | $CH_3$ | $C_2H_5$ | H | $CO_2CH_3$ | $CH_3$ | |
| 4 | $CH_3$ | $CH_3$ | H | $CO_2CH_3$ | $C_2H_5$ | |
| 5 | $CH_3$ | $C_2H_5$ | H | $CO_2CH_3$ | $C_2H_5$ | |
| 6 | $C_2H_5$ | $C_2H_5$ | H | $CO_2CH_3$ | $C_2H_5$ | |
| 7 | $CH_3$ | $CH_3$ | H | $CO_2CH_3$ | $CH(CH_3)_2$ | |
| 8 | $C_2H_5$ | $C_2H_5$ | H | $CO_2CH_3$ | $CH(CH_3)_2$ | 1,516 |
| 9 | $CH_3$ | $CH_3$ | H | $CO_2CH_3$ | $CH(Cl)CH_3$ | 100 |
| 10 | $CH_3$ | $C_2H_5$ | H | $CO_2CH_3$ | $CH(Cl)CH_3$ | |
| 11 | $CH_3$ | $CH_3$ | H | $COSCH_3$ | $CH(Cl)CH_3$ | |
| 12 | $CH_3$ | $CH_3$ | H | CN | $CH(Cl)CH_3$ | |
| 13 | $CH_3$ | $CH_3$ | H | $CO_2CH_3$ | $CH_2-CH_2Cl$ | Öl |
| 14 | $CH_3$ | $CH_3$ | H | $CO_2CH_3$ | $CH_2-CH_2-CH_2Cl$ | |
| 15 | $CH_3$ | $CH_3$ | H | $CO_2CH_3$ | $CHCl_2$ | |
| 16 | $CH_3$ | $C_2H_5$ | H | $CO_2CH_3$ | $CHCl_2$ | |
| 17 | $CH_3$ | $CH_3$ | H | $CO_2C_2H_5$ | $CHCl_2$ | |
| 18 | $CH_3$ | $CH_3$ | H | $CO_2i-C_3H_7$ | $CHCl_2$ | |
| 19 | $CH_3$ | $CH_3$ | H | $COSCH_3$ | $CHCl_2$ | |
| 20 | $CH_3$ | $CH_3$ | H | $CO_2CH_2$ | $CCl_3$ | |
| 21 | $CH_3$ | $CH_3$ | H | $CO_2CH_3$ | $CH=CH_2$ | 92 |
| 22 | $CH_3$ | $CH_3$ | H | $CO_2CH_3$ | $C\equiv CH$ | |
| 23 | $CH_3$ | $CH_3$ | H | $CO_2CH_3$ | $\underset{CH_3}{\underset{\mid}{C}}=CH_2$ | 90 |
| 24 | $CH_3$ | $CH_3$ | H | $CO_2CH_3$ | $\underset{Cl}{\underset{\mid}{C}}=CH_2$ | |
| 25 | $CH_3$ | $CH_3$ | H | $CO_2CH_3$ | $CH=CCl_2$ | |
| 26 | $CH_3$ | $CH_3$ | H | $CO_2C_2H_5$ | $CCl=CCl_2$ | |
| 27 | $CH_3$ | $CH_3$ | H | $CO_2CH_3$ | 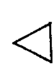 | 106 |

| Wirkstoff-Nr. | $R^1$ | $R^2$ | $R^3$ | $R^4$ | $R^5$ | Fp. (°C) oder $n_D^{20}$ |
|---|---|---|---|---|---|---|
| 28 | $CH_3$ | $C_2H_5$ | H | $CO_2CH_3$ | 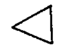 | |
| 29 | $C_2H_5$ | $C_2H_5$ | H | $CO_2CH_3$ | 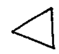 | |
| 30 | $CH_3$ | H | 3-$CH_3$ | $CO_2C_2H_5$ | 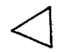 | |
| 31 | $CH_3$ | H | 5-$CH_3$ | $CO_2C_2H_5$ | 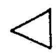 | |
| 32 | $CH_3$ | H | 5-Cl | $CO_2CH_3$ | 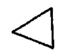 | |
| 33 | $CH_3$ | $CH_3$ | H | $COSCH_3$ | 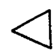 | |
| 34 | $CH_3$ | $CH_3$ | H | $CO_2CH_3$ | 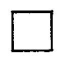 | |
| 35 | $CH_3$ | $CH_3$ | H | $CO_2CH_3$ | 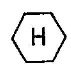 | |
| 36 | $C_2H_5$ | $C_2H_5$ | H | $CO_2CH_3$ | 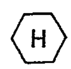 | viskos. |
| 37 | $CH_3$ | $CH_3$ | H | $CO_2CH_3$ | $CH_2OCH_3$ | 74–76 |
| 38 | $CH_3$ | $CH_3$ | 6-$CH_3$ | $CO_2CH_3$ | $CH_2OCH_3$ | |
| 39 | $C_2H_5$ | $C_2H_5$ | H | $CO_2CH_3$ | $CH_2OCH_3$ | |
| 40 | $CH_3$ | $CH_3$ | H | CN | $CH_2OCH_3$ | |
| 41 | $CH_3$ | $CH_3$ | H | $CO_2C_2H_5$ | $CH_2OCH_3$ | 86 |
| 42 | $CH_3$ | $CH_3$ | H | $CO_2i\text{-}C_3H_7$ | $CH_2OCH_3$ | |
| 43 | $CH_3$ | $CH_3$ | H | $COSCH_3$ | $CH_2OCH_3$ | 118 |
| 44 | $CH_3$ | H | 5-$CH_3$ | $CO_2CH_3$ | $CH_2OCH_3$ | |
| 45 | $CH_3$ | H | 4-Cl | $CO_2CH_3$ | $CH_2OCH_3$ | 103 |
| 46 | $CH_3$ | H | 4-$CH_3O$ | $CO_2CH_3$ | $CH_2OCH_3$ | |
| 47 | $CH_3$ | $CH_3$ | H | $CO_2CH_3$ | $CH_2SCH_3$ | |
| 48 | $CH_3$ | $C_2H_5$ | H | $CO_2CH_3$ | $CH_2SCH_3$ | |
| 49 | $C_2H_5$ | $C_2H_5$ | H | $CO_2CH_3$ | $CH_2SCH_3$ | |
| 50 | $CH_3$ | $CH_3$ | H | $COSCH_3$ | $CH_2SCH_3$ | |
| 51 | $CH_3$ | $CH_3$ | H | $CO_2CH_3$ | $CH_2\text{-}CN$ | |
| 52 | $CH_3$ | $CH_3$ | H | $COSCH_3$ | 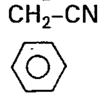 | |
| 53 | $CH_3$ | $CH_3$ | H | $CO_2CH_3$ | 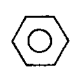 | 136 |
| 54 | $CH_3$ | $CH_3$ | H | CN | 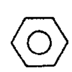 | |
| 55 | $CH_3$ | $CH_3$ | H | $CO_2CH_3$ | 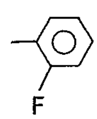 | 117 |
| 56 | $CH_3$ | $CH_3$ | H | $CO_2CH_3$ | 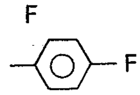 | 145 |

| Wirkstoff-Nr. | $R^1$ | $R^2$ | $R^3$ | $R^4$ | $R^5$ | Fp. (°C) oder $n_D^{20}$ |
|---|---|---|---|---|---|---|
| 57 | $CH_3$ | $CH_3$ | H | $CO_2CH_3$ | 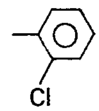 | Öl |
| 58 | $CH_3$ | $CH_3$ | H | $CO_2CH_3$ | 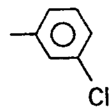 | 98–101 |
| 59 | $CH_3$ | $CH_3$ | H | $CO_2CH_3$ | 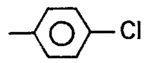 | 161 |
| 60 | $CH_3$ | $CH_3$ | H | $CO_2CH_3$ | 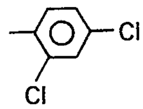 | 90 |
| 61 | $CH_3$ | H | 5–$CH_3$ | $CO_2CH_3$ | 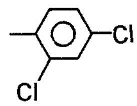 | |
| 62 | $CH_3$ | $CH_3$ | H | $COSCH_3$ | 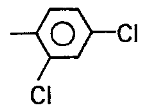 | |
| 63 | $CH_3$ | $CH_3$ | H | $CO_2CH_3$ | 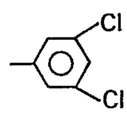 | 139 |
| 64 | $CH_3$ | $CH_3$ | H | $CO_2CH_3$ | 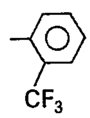 | |
| 65 | $CH_3$ | $CH_3$ | H | $CO_2CH_3$ | 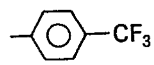 | |
| 66 | $C_2H_5$ | $C_2H_5$ | H | $CO_2CH_3$ | 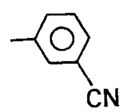 | |
| 67 | $CH_3$ | $CH_3$ | H | $CO_2CH_3$ | 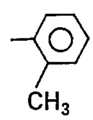 | 105–106 |
| 68 | $CH_3$ | $CH_3$ | H | $CO_2CH_3$ | 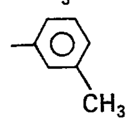 | 125–27 |
| 69 | $CH_3$ | $CH_3$ | H | $CO_2CH_3$ | 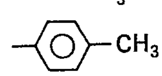 | 117–119 |
| 70 | $CH_3$ | $CH_3$ | H | $CO_2CH_3$ | 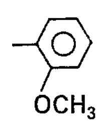 | |
| 71 | $CH_3$ | $CH_3$ | H | $CO_2CH_3$ | 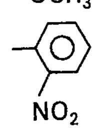 | |

| Wirkstoff-Nr. | $R^1$ | $R^2$ | $R^3$ | $R^4$ | $R^5$ | Fp. (°C) oder $n_D^{20}$ |
|---|---|---|---|---|---|---|
| 72 | $CH_3$ | $CH_3$ | H | $CO_2CH_3$ | 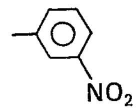 | 132–133 |
| 73 | $CH_3$ | $CH_3$ | H | $CO_2CH_3$ | 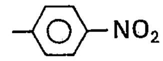 | |
| 74 | $CH_3$ | $CH_3$ | H | $CO_2CH_3$ | 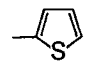 | 115–116 |
| 75 | $CH_3$ | $CH_3$ | H | $CO_2C_2H_5$ | 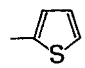 | |
| 76 | $CH_3$ | $C_2H_5$ | H | $CO_2C_2H_5$ | 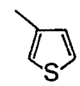 | |
| 77 | $CH_3$ | $CH_3$ | H | $CO_2CH_3$ | 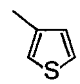 | 121 |
| 78 | $CH_3$ | $CH_3$ | H | $CO_2CH_3$ | 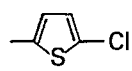 | |
| 79 | $CH_3$ | $CH_3$ | H | $CO_2CH_3$ | 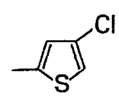 | 130–32 |
| 80 | $CH_3$ | $CH_3$ | H | $CO_2CH_3$ | 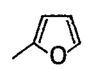 | 96–98 |
| 81 | $CH_3$ | $C_2H_5$ | H | $CO_2C_2H_5$ | 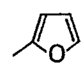 | Öl |
| 82 | $CH_3$ | $CH_3$ | H | $COSCH_3$ | 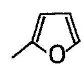 | 121 |
| 83 | $CH_3$ | $CH_3$ | H | CN | 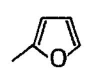 | |
| 84 | $CH_3$ | H | 5–$CH_3$ | $CO_2CH_3$ | 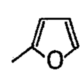 | |
| 85 | $C_2H_5$ | $C_2H_5$ | H | $CO_2CH_3$ | 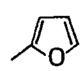 | 101–103 |
| 86 | $CH_3$ | $CH_3$ | $CH_3$ | $CO_2CH_3$ | 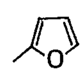 | |
| 87 | $CH_3$ | $CH_3$ | H | $CO_2C_2H_5$ | 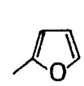 | Öl |
| 88 | $CH_3$ | $CH_3$ | H | $CO_2i–C_3H_7$ | 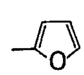 | |
| 89 | $CH_3$ | $CH_3$ | H | $CO_2CH_3$ | 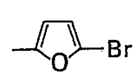 | |
| 90 | $CH_3$ | $CH_3$ | H | $CO_2CH_3$ | 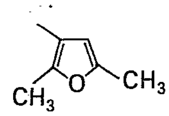 | 126–28 |

| Wirkstoff-Nr. | $R^1$ | $R^2$ | $R^3$ | $R^4$ | $R^5$ | Fp. (°C) oder $n_D^{20}$ |
|---|---|---|---|---|---|---|
| 91 | $CH_3$ | $CH_3$ | H | $CO_2CH_3$ | 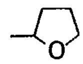 | |
| 92 | $CH_3$ | $CH_3$ | H | $CO_2CH_3$ | 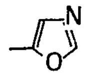 | |
| 93 | $CH_3$ | $CH_3$ | H | $CO_2CH_3$ | 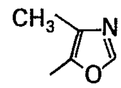 | 122 |
| 94 | $CH_3$ | $CH_3$ | H | $CO_2CH_3$ | 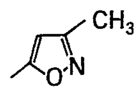 | 106–08 |
| 95 | $CH_3$ | $CH_3$ | H | $CO_2CH_3$ | 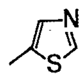 | |
| 96 | $CH_3$ | H | 4–Cl | $CO_2CH_3$ | 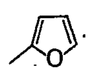 | |
| 97 | $CH_3$ | H | H | $CO_2CH_3$ | 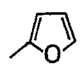 | |
| 98 | H | $CH_3$ | 3–Cl | $CO_2CH_3$ | 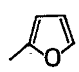 | |
| 99 | H | Cl | H | $CO_2CH_3$ | 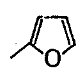 | |
| 100 | $CH_3$ | H | 5–Cl | $CO_2CH_3$ | $CH_2OCH_3$ | 95 |
| 101 | $CH_3$ | H | H | $CO_2CH_3$ | $CH_2OCH_3$ | 90 |
| 102 | H | Cl | H | $CO_2CH_3$ | $CH_2OCH_3$ | |
| 103 | $CH_3$ | $C_2H_5$ | H | $CO_2CH_3$ | $CH_2OCH_3$ | 1,5261 |
| 104 | $CH_3$ | $C_2H_5$ | H | $CO_2C_2H_5$ | $CH_2OCH_3$ | 1,5210 |
| 105 | $CH_3$ | $CH_3$ 4–t. | $C_4H_9$ | $CO_2CH_3$ | $CH_2OCH_3$ | 98 |
| 106 | $CH_3$ | H 5–t | $C_4H_9$ | $CO_2CH_3$ | $CH_2OCH_3$ | 1,5130 |
| 107 | $CH_3$ | $CH_3$ | H | $CO_2CH_3$ | $CH_2-CH_2OCH_3$ | Öl |
| 108 | $CH_3$ | $CH_3$ | H | $CO_2C_2H_5$ | $CH_2-CH_2OCH_3$ | Öl |
| 109 | $CH_3$ | $CH_3$ | H | $CO_2CH_3$ | $CH(CH_3)OCH_3$ | Öl |
| 110 | $CH_3$ | $CH_3$ | H | $CO_2C_2H_5$ | $CH(CH_3)OCH_3$ | 84–86 |
| 111 | $CH_3$ | $CH_3$ | H | $CO_2CH_2CH{=}CH_2$ | $CH_2OCH_3$ | Öl |
| 112 | $CH_3$ | $CH_3$ | H | $CO_2CH_2CH{=}CH_2$ | 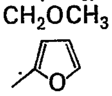 | |
| 113 | $CH_3$ | $CH_3$ | H | $CO_2CH_3$ | $CH_2Cl$ | 125–28 |
| 114 | $CH_3$ | $C_2H_5$ | H | $CO_2CH_3$ | $CH_2Cl$ | 61–63 |
| 115 | $CH_3$ | $CH_3$ | H | $CO_2C_2H_5$ | $CH_2Cl$ | 93–95 |
| 116 | $CH_3$ | $CH_3$ | H | $CO_2CH_3$ | $CH_2-CH_2-F$ | |
| 117 | $CH_3$ | $CH_3$ | H | $CO_2CH_3$ | 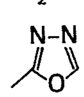 | |
| 118 | $CH_3$ | $CH_3$ | H | $CO_2CH_3$ | 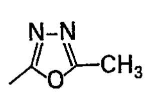 | |
| 119 | $CH_3$ | $CH_3$ | H | $CO_2CH_3$ | 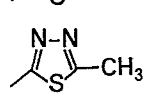 | |

| Wirkstoff-Nr. | $R^1$ | $R^2$ | $R^3$ | $R^4$ | $R^5$ | Fp. (°C) oder $n_D^{20}$ |
|---|---|---|---|---|---|---|
| 120 | $CH_3$ | $CH_3$ | H | $CO_2CH_3$ | 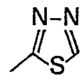 | |
| 121 | $CH_3$ | $CH_3$ | H | $CO_2CH_3$ | 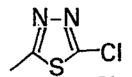 | |
| 122 | $CH_3$ | $CH_3$ | H | $CO_2CH_3$ | 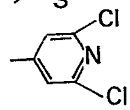 | 143–144 |
| 123 | $CH_3$ | $CH_3$ | H | $CO_2C_2H_5$ | 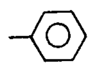 | 110 |
| 124 | $CH_3$ | $CH_3$ | H | $CO_2CH_3$ | 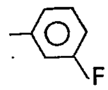 | 136 |
| 125 | $CH_3$ | $CH_3$ | H | $CO_2C_2H_5$ | 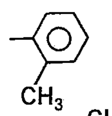 | 123–24 |
| 126 | $CH_3$ | $CH_3$ | H | $CO_2C_2H_5$ | 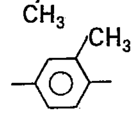 | 95–97 |
| 127 | $CH_3$ | $CH_3$ | H | $CO_2C_2H_5$ | 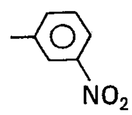 | 193 |
| 128 | $CH_3$ | $CH_3$ | H | $CO_2CH_3$ | 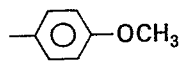 | 102–04 |
| 129 | $CH_3$ | $CH_3$ | H | $CO_2CH_3$ | 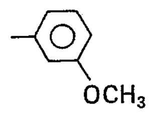 | 105–06 |
| 130 | $CH_3$ | $CH_3$ | H | $CO_2CH_3$ | 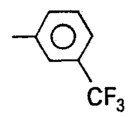 | 108–09 |
| 131 | $CH_3$ | $CH_3$ | H | $CO_2CH_3$ | 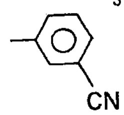 | 145–47 |
| 132 | $CH_3$ | $CH_3$ | H | $CO_2CH_3$ | $CH_2OC_2H_5$ | 76–78 |
| 133 | $CH_3$ | $CH_3$ | H | $CO_2C_2H_5$ | $CH_2OC_2H_5$ | 79 |
| 134 | $CH_3$ | $CH_3$ | H | $CO_2CH_3$ | 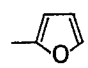 | Öl |
| 135 | $CH_3$ | $CH_3$ | H | $CO_2C_2H_5$ | 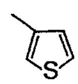 | 64–68 |
| 136 | $CH_3$ | $CH_3$ | H | $CO_2CH_3$ | 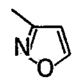 | 99 |

Die neuen Wirkstoffe werden beispielsweise in Form von direkt versprühbaren Lösungen, Pulvern, Suspensionen, auch hochprozentige wässrige, ölige oder sonstige Suspensionen oder Dispersionen, Emulsionen, Öldispersionen, Pasten, Stäubemitteln, Streumitteln, Granulaten durch

Versprühen, Vernebeln, Verstäuben, Verstreuen oder Giessen angewendet. Die Anwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wässrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht: Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutynaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther, Tributylphenylpolyglykoläther, Alkalarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Atteclay, Kalkstein, Kreide, Talkum, Bolus, Löss, Ton, Dolomit, Diatomeenerde, Calcium- und Magnesiumsulfat, Magnesiumoxid gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nusschalenmehl, Cellulosepulver und andere feste Trägerstoffe.

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cyctorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben. Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen. Darüberhinaus sind viele der neuen Verbindungen systemisch wirksam, so dass über die Wurzelbehandlung auch ein Schutz oberirdischer Pflanzenteile möglich ist.

Ferner lassen sich mit den neuen Verbindungen auch Pilze, die Keimlings- und Auflaufkrankheiten hervorrufen, beispielsweise Pythium- und Aphamomyces-Arten an Leguminosen und Baumwolle, bekämpfen. Die Aufwandmengen betragen je 100 kg Saatgut 10 bis 200 g Wirkstoff; die Anwendung erfolgt in Form von Saatgutbeizmitteln.

Die neuen Wirkstoffe können auch zusammen mit anderen Wirkstoffen, z.B. Herbiziden, Insektiziden, Wachstumsregulatoren und Fungiziden oder auch mit Düngemitteln vermischt und ausgebracht werden. In vielen Fällen erhält man bei der Mischung mit Fungiziden eine Vergrösserung des fungiziden Wirkungsspektrums; bei einer Anzahl dieser Fungizidmischungen treten auch synergistische Effekte auf, d.h. die fungizide Wirksamkeit des Kombinationsproduktes ist grösser als die der addierten Wirksamkeiten der Einzelkomponenten. Eine besonders günstige Vergrösserung des Wirkungsspektrums wird mit folgenden Fungiziden erzielt:

Manganäthylenbisdithiocarbamat

Mangan-Zinkäthylenbisdithiocarbamat

Ammoniak-Komplex von Zink-(N,N-äthylen-bis-

dithiocarbamat)
N-Trichlormethylthio-tetrahydcrophthalimid
N-Trichlormethyl-phthalimid
5-Äthoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
5-Methyl-5-vinyl-3-(3,5-dichlorphenyl)-2,4-dioxo-1,3-oxazolidin
5-Methyl-5-methoxymethyl-3-(3,5-dichlorphenyl)-2,4-dioxo-1,3-oxazolidin-N-p-Fluorphenyl-2,3-dichlormaleinimid

Die folgende Liste von Fungiziden, mit denen die erfindungsgemässen Verbindungen kombiniert werden können, soll die Kombinationsmöglichkeiten erläutern, nicht aber einschränken.

Fungizide, die mit den erfindungsgemässen Wirkstoffen kombiniert werden können, sind beispielsweise:

Dithiocarbamate und deren Derivate, wie
Ferridimethyldithiocarbamat,
Zinkdimethyldithiocarbamat,
Zinkäthylenbisdithiocarbamat,
Tetramethylthiuramidsulfide,
Zink-(N,N-propylen-bis-dithiocarbamat),
Ammoniak-Komplex von Zink(N,N'-propylen-bis-dithiocarbamat) und
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethyl-acrylat,
2-sec-Butyl-4,6-dinitrophenyl-isopropyl-carbonat;

heterocyclische Strukturen, wie
2-Heptadecyl-2-imidazolin-acetat,
2,4-Dichlor-6-(o-chloranilino)-s-triazin,
0,0-Diäthyl-phthalimidophonothioat,
5-Amino-1-(bis-(dimethylamino)-phosphinyl)-3-phenyl-1,2,4-triazol),
2,3-Dicyano-1,4-dithiaanthrachinon,
2-Thio-1-1,3-dithio-(4,5-b)-chinoxalin,
1-(Butylcarbamoyl)-2-benzimidazol-carbaminsäuremethyl-ester,
4-(2-Chlorphenylhydrazono)-3-methyl-5-isocazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid
2,3-Dihydro-5-carboxanilido-6-methyl-1,4-oxathiin,
2-(Furyl-(2))-benzimidazol,
Piperazin-1,4-diylbis-(1-(2,2,2-trichlor-äthyl)-formamid, 2-(Thiazolyl-(4)-benzimidazol,
5-Butyl-2-dimethylamino-4-hydroxy-6-methyl-pyrimidin,
Bis-(p-Chlorphenyl)-3-pyridinmethanol,
1,2-Bis-(3-äthoxycarbonyl-2-thioureido)-benzol,
1,2-Bis-(3-methoxycarbonyl-2-thioureido)-benzol,

sowie verschiedene Fungizide, wie
Dodecylguanidinacetat,
3-(3-(3,5-Dimethyl-2-oxycyclohexyl)-2-hydroxyäthyl)-gluarimid,
Hexachlorbenzol,
N-Dichlorfluormethylthio-N,N'-dimethyl-N-phenyl-schwefelsäurediamid,
2,5-Dimethyl-furan-3-carbonsäureanilid,
2-Methyl-benzoesäure-anilid,
2-Jod-benzoesäure-anilid,
1-(3,4-Dichloranilino)-1-formylamino-2,2,2-trichloräthan,
2,6-Diumethyl-N-tridecyl-morpholin bzw. dessen Salze,
2,6-Dimethyl-N-cyclododecyl-morpholin bzw. dessen Salze.
α-(2-Chlorphenyl)-α-(4-chlorphenyl)-5-pyrimidin-methanol,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol.

Die folgenden Beispiele erläutern die fungizide Wirkung der Verbindungen. Als Vergleichssubstanzen werden in den Beispielen folgende bekannte fungizide Verbindungen angeführt:

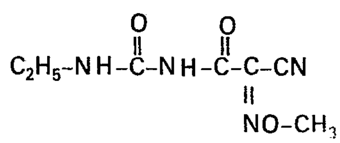

Verbindung A

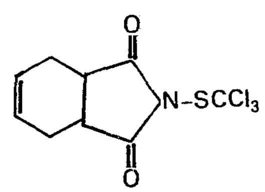

Verbindung B

Beispiel 4

Fungizide Wirksamkeit gegen Phytophthora infestans an Tomaten.

Blätter von Tomatenpflanzen der Sorte «Professor Rudloff» werden mit wässrigen Suspensionen, die 80% (Gewichtsprozent) des zu prüfenden Wirkstoffes und 20% Natriumligninsulfonat in der Trockensubstanz enthalten, besprüht. Es werden 0,025 und 0,012%ige Spritzbrühen (berechnet auf die Trockensubstanz) verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten, jedoch infizierten Kontrollpflanzen so

stark entwickelt, dass die fungizide Wirksamkeit der Substanzen beurteilt werden kann:

0 = kein Pilzbefall, abgestuft bis 5 = Totalbefall (Kontrolle)

| Wirkstoff | Befall der Blätter nach Spritzung mit 00%iger Wirkstoffbrühe | |
|---|---|---|
| | 0,025 | 0,012 |
| 37 | 0 | 1 |
| 56 | 0 | 0 |
| 74 | 0 | 0 |
| 80 | 0 | 1 |
| A (bekannt) | 2 | 1 |
| Kontrolle (unbehandelt) | 5 | |

Beispiel 5

Fungizide Wirksamkeit gegen Plasmopara viticola an Reben

Blätter von Topfreben der Sorte Müller-Thurgau werden mit einer Zoosporenaufschwemmung des Pilzes Plasmopara viticola infiziert. Die Pflanzen kommen dann für 16 Stunden in eine wasserdampfgesättigte (feuchte) Kammer bei 24°C. Während dieser Zeit erfolgt die Infektion des Blattgewebes. Danach werden die Pflanzen zur Ermittlung der kurativen Wirksamkeit mit wässrigen Suspensionen, die 80% (Gewichtsprozent) des zu prüfenden Wirkstoffes und 20% Natriumligninsulfonat in der Trockensubstanz enthalten, besprüht. Es werden 0,05% Spritzbrühen (bezogen auf die Trockensubstanz) verwendet. Nach der Wirkstoffanwendung werden die Reben 8 Tage im Gewächshaus zwischen 22 und 24°C aufgestellt. Nach dieser Zeit werden die Pflanzen zur Beschleunigung und Verstärkung des Sporangienträgerausbruches abermals während 16 Stunden in der feuchten Kammer aufgestellt. Dann wird eine Beurteilung des Krankheitsausbruches vorgenommen.

0 = kein Pilzbefall, abgestuft bis 5 = Totalbefall (Kontrolle)

| Wirkstoff | Befall der Blätter nach Spritzung mit 0,05%iger Wirkstoffbrühe |
|---|---|
| 1 | 0 |
| 8 | 0 |
| 9 | 0 |
| 27 | 0 |
| 36 | 0 |
| 37 | 0 |
| 53 | 0 |
| 80 | 0 |
| 85 | 0 |
| A (bekannt) | 2–3 |
| Kontrolle (unbehandelt) | 5 |

Beispiel 6

Fungizide Wirksamkeit gegen Auflaufkrankheiten an Erbsen

100-g-Proben Erbsensamen der Sorte «Senator» werden in Glasflaschen etwa 5 Minuten lang mit 300 mg (= 0,3 Gew.-%) Beizmittelaufbereitungen, die 40% Wirkstoff und 60% Ton in der Trokkensubstanz enthalten, sorgfältig geschüttelt. Danach werden jeweils 100 Samen in Saatkisten 3 cm tief und mit einem Abstand von 3 bis 5 cm in eine Komposterde eingesät, die eine starke natürliche Verseuchung mit den Pilzen Pythium spec., Aphanomyces spec. und Fusarium oxysporum aufweist. Die Kästen werden im Gewächshaus bei Temperaturen von 17 bis 20°C aufgestellt. Nach einer Versuchsdauer von 21 Tagen wird die Anzahl gesunder Erbsenpflanzen ermittelt.

| Wirkstoff | 00% gesunde Pflanzen nach 21 Tagen in Komposterde |
|---|---|
| 74 | 90 |
| B (bekannt) | 60 |
| Kontrolle (unbehandelt) | 10 |
| Kontrolle sterilisierte Komposterde | 93 |

Beispiel 7

Man vermischt 90 Gewichtsteile der Verbindung 1 mit 10 Gewichtsteilen N-Methyl-pyrrolidon und erhält eine Mischung, die zur Anwendung in Form kleinster Tropfen geeignet ist.

Beispiel 8

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgiessen und feines Verteilen der Lösung in 100000 Gewichtsteilen Wasser erhält man eine wässrige Dispersion, die 0,02 Gewichtsprozent des Wirkstoffs enthält.

Beispiel 9

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingiessen und feines Verteilen der Lösung in 100000 Gewichtsteilen Wasser erhält

man eine wässrige Dispersion, die 0,02 Gewichtsprozent des Wirkstoffs enthält.

Beispiel 10

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Beispiel 11

20 Gewichtsteile des Wirkstoffs 2 werden mit 3 Gewichtsteilen des Natriumsalzes der Diisobutylnaphthalin- -sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 12

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 13

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 14

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gewichtsprozent Wirkstoff enthält.

Beispiel 15

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche**

Fassung A für die Vertragsstaaten BE, CH, DE, FR, GB, IT, LU, NL und SF

1. Substituierte Cyanamide der allgemeinen Formel

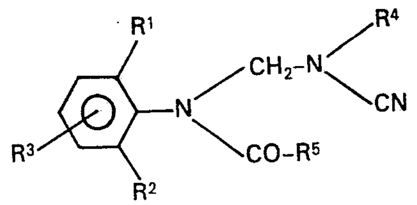

I

in welcher

$R^1$ $C_1$–$C_4$ Alkyl oder $C_1$–$C_4$-Alkoxy,

$R^2$ Wasserstoff, $C_1$–$C_4$ Alkyl, $C_1$–$C_4$ Alkoxy oder Halogen,

$R^3$ Wasserstoff, $C_1$–$C_4$-Alkyl oder Halogen,

$R^4$ $C_1$–$C_6$-Alkoxcarbonyl, $C_3$–$C_4$-Alkenoxycarbonyl, $C_1$–$C_6$-Alkyl-thiocarbonyl oder Cyan und

$R^5$ ein gegebenenfalls durch niederes Alkoxy, Alkylthio, Cyan oder Halogen substituiertes $C_1$–$C_6$ Alkyl, ein gegebenenfalls durch Halogen substituiertes $C_2$–$C_5$ Alkenyl, ein $C_2$–$C_4$ Alkinyl, ein $C_3C_7$ Cycloalkyl, einen gegebenenfalls durch niederes Alkyl oder Halogen substituierten heterocyclischen Rest bedeutet, der ein Sauerstoffatom oder ein Schwefelatom oder ein Stickstoffatom oder bis zu zwei Stickstoffatome und ein Sauerstoffatom oder bis zu zwei Stickstoffatome und ein Schwefelatom enthält, $R^5$ ferner ein Phenyl oder ein durch ein bis drei gleiche oder verschiedene Substituenten aus der Gruppe Fluor, Chlor, Brom, Trifluormethyl, Cyan, Nitro, $C_1$–$C_4$-Alkyl, $C_1$–$C_4$ Alkoxy substituiertes Phenyl bedeutet.

2. Fungizid, enthaltend als eine Komponente ein substituiertes Cyanamid gemäss Anspruch 1.

3. Fungizid gemäss Anspruch 2, enthaltend einen festen oder flüssigen Trägerstoff und ein substituiertes Cyanamid gemäss Anspruch 1.

4. Verwendung eines substituierten Cyanamids gemäss Anspruch 1 zur Bekämpfung von Pilzen oder Pilzbefall.

5. Verfahren zur Herstellung eines substituierten Cyanamids gemäss Anspruch 1, dadurch gekennzeichnet, dass man ein Mol eines N-Halogenmethylanilids der Formel

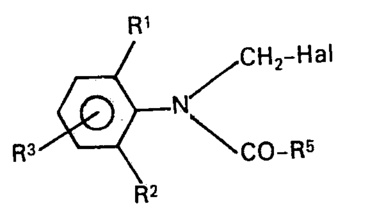

II

in welcher $R^1$, $R^2$, $R^3$ und $R^5$ die oben angegebene Bedeutung haben und Hal Halogen, insbesondere Chlor oder Brom bedeutet, mit mindestens einem Mol eines Cyanamids der Formel

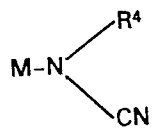

III

in welcher $R^4$ die oben angegebene Bedeutung hat und M Wasserstoff, ein Alkalimetall oder einen Tetraalkylammonium-Rest bedeutet, bei einer Temperatur zwischen 0 und 150 °C, gegebenenfalls in Gegenwart eines Säurebindemittels und gegebenenfalls in Gegenwart eines gegenüber den Reaktionspartnern inerten Lösungsmittels umsetzt.

6. Verfahren zur Herstellung eines substituierten Cyanamids gemäss Anspruch 5, dadurch gekennzeichnet, dass man die Umsetzung in einem Zweiphasensystem, wobei die eine Phase Wasser und die andere Phase ein mit der wässrigen Phase nicht mischbares gegenüber den Reaktionspartnern inertes Lösungsmittel ist, in Gegenwart von 0,5 bis 30 Molprozent eines Phasentransferkatalysators durchführt.

7. Substituiertes Cyanamid gemäss Anspruch 1, nämlich
N-Methoxycarbonyl-N-(N'methoxyacetyl, N'-2',6'-dimethylanilinomethyl)-cyanamid
N-Äthoxycarbonyl-N-(N'-methoxyacetyl, N'-2',6'-dimethylanilinomethyl)-cyanamid
N-Methoxycarbonyl-N-(N'-3-fluorbenzoyl, N'-2',6'-dimethylanilinomethyl)-cyanamid
N-Methoxycarbonyl-N-(N'-4-fluorbenzoyl, N'-2',6'-dimethylanilinomethyl)-cyanamid
N-Methoxycarbonyl-N-(N'-furo-2-yl N'-2',6'-dimethylanilinomethyl)-cyanamid
N-Äthoxycarbonyl-N-(N'-furo-2-yl, N'-2',6'-dimethylanilinomethyl)-cyanamid, oder
N-Methoxycarbonyl-N-(N'-theno-2-yl, N'-2',6'-dimethylanilinomethyl)-cyanamid.

8. Fungizid gemäss Anspruch 2, enthaltend ein substituiertes Cyanamid, nämlich
N-Methoxycarbonyl-N-(N'-methoxyacetyl, N'-2',6'-dimethylanilinomethyl)-cyanamid
N-Äthoxycarbonyl-N-(N'-methoxyacetyl, N'-2',6'-dimethylanilinomethyl)-cyanamid
N-Methoxycarbonyl-N-(N'-3-fluorbenzoyl, N'-2',6'-dimethylanilinomethyl)-cyanamid
N-Methoxycarbonyl-N-(N'-4-fluorbenzoyl, N'-2',6'-dimethylanilinomethyl)-cyanamid
N-Methoxycarbonyl-N-(N'-furo-2-yl N'-2',6'-dimethylanilinomethyl)-cyanamid
N-Äthoxycarbonyl-N-(N'-furo-2-yl, N'-2',6'-dimethylanilinomethyl)-cyanamid, oder
N-Methoxycarbonyl-N-(N'-theno-2-yl, N'-2',6'-dimethylanilinomethyl)-cyanamid.

## Patentansprüche

Fassung B für Vertragsstaat AT

1. Fungizid, enthaltend als eine Komponente ein substituiertes Cyanamid der allgemeinen Formel

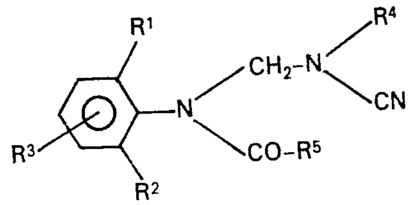

I

in welcher
$R^1$ $C_1$-$C_4$ Alkyl oder $C_1$-$C_4$-Alkoxy,
$R^2$ Wasserstoff, $C_1$-$C_4$ Alkyl, $C_1$-$C_4$ Alkoxy oder Halogen,
$R^3$ Wasserstoff, $C_1$-$C_4$-Alkyl oder Halogen,
$R^4$ $C_1$-$C_6$-Alkoxcarbonyl, $C_3$-$C_4$-Alkenoxycarbonyl, $C_1$-$C_6$-Alkyl-thiocarbonyl oder Cyan und
$R^5$ ein gegebenenfalls durch
niederes Alkoxy, Alkylthio, Cyan oder Halogen substituiertes $C_1$-$C_6$ Alkyl, ein gegebenenfalls durch
Halogen substituiertes $C_2$-$C_5$ Alkenyl, ein $C_2$-$C_4$ Alkinyl, ein $C_3C_7$ Cycloalkyl, einen gegebenenfalls durch niederes Alkyl oder Halogen substituierten heterocyclischen Rest bedeutet, der ein Sauerstoffatom oder ein Schwefelatom oder ein Stickstoffatom oder bis zu zwei Stickstoffatome und ein Sauerstoffatom oder bis zu zwei Stickstoffatome und ein Schwefelatom enthält, $R^5$ ferner ein Phenyl oder ein durch ein bis drei gleiche oder verschiedene Substituenten aus der Gruppe Fluor, Chlor, Brom, Trifluormethyl, Cyan, Nitro, $C_1$-$C_4$-Alkyl, $C_1$-$C_4$ Alkoxy substituiertes Phenyl bedeutet.

2. Fungizid nach Anspruch 1, enthaltend einen festen oder flüssigen Trägerstoff und ein substituiertes Cyanamid wie in Anspruch 1 definiert.

3. Verwendung eines substituierten Cyanamids wie in Anspruch 1 definiert zur Bekämpfung von Pilzen oder Pilzbefall.

4. Verfahren zur Herstellung eines substituierten Cyanamids wie in Anspruch 1 definiert, dadurch gekennzeichnet, dass man ein Mol eines N-Halogenmethylanilids der Formel

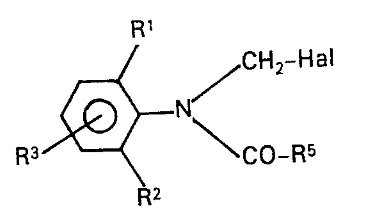

II

in welcher $R^1$, $R^2$, $R^3$ und $R^5$ die oben angegebene Bedeutung haben und Hal Halogen, insbesondere Chlor oder Brom bedeutet, mit mindestens einem Mol eines Cyanamids der Formel

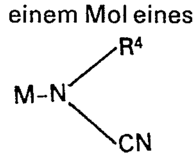

III

in welcher $R^4$ die oben angegebene Bedeutung hat und M Wasserstoff, ein Alkalimetall oder einen Tetraalkylammonium-Rest bedeutet, bei einer Temperatur zwischen 0 und 150 °C, gegebenenfalls in Gegenwart eines Säurebindemittels

und gegebenenfalls in Gegenwart eines gegenüber den Reaktionspartnern inerten Lösungsmittels umsetzt.

5. Verfahren nach Anspruch 4, dadurch gekennzeichnet, dass man die Umsetzung in einem Zweiphasensystem, wobei die eine Phase Wasser und die andere Phase ein mit der wässrigen Phase nicht mischbares gegenüber den Reaktionspartnern inertes Lösungsmittel ist, in Gegenwart von 0,5 bis 30 Molprozent eines Phasentransferkatalysators durchführt.

6. Fungizid nach Anspruch 1, enthaltend ein substituiertes Cyanamid, nämlich
N-Methoxycarbonyl-N-(N'-methoxyacetyl, N'-2',6'-dimethylanilinomethyl)-cyanamid
N-Äthoxycarbonyl-N-(N'-methoxyacetyl, N'-2',6'-dimethylanilinomethyl)-cyanamid
N-Methoxycarbonyl-N-(N'-3-fluorbenzoyl, N'-2',6'-dimethylanilinomethyl)-cyanamid
N-Methoxycarbonyl-N-(N'-4-fluorbenzoyl, N'-2',6'-dimethylanilinomethyl)-cyanamid
N-Methoxycarbonyl-N-(N'-furo-2-yl N'-2',6'-dimethylanilinomethyl)-cyanamid
N-Äthoxycarbonyl-N-(N'-furo-2-yl, N'-2',6'-dimethylanilinomethyl)-cyanamid
N-Methoxycarbonyl-N-(N'-theno-2-yl, N'-2',6'-dimethylanilinomethyl)-cyanamid

**Claims**

for the Contracting states: BE, CH, DE, FR, GB, IT, LU, NL and SE

1. A substituted cyanamide of the general formula

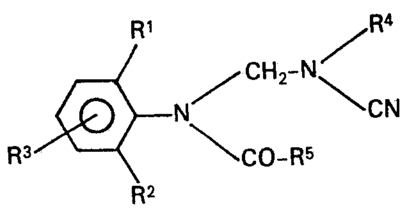

I

where
$R^1$ denotes $C_1$–$C_4$-alkyl or $C_1$–$C_4$-alkoxy,
$R^2$ denotes hydrogen, $C_1$–$C_4$-alkyl, $C_1$–$C_4$-alkoxy or halogen,
$R^3$ denotes hydrogen, $C_1$–$C_4$-alkyl or halogen,
$R^4$ denotes $C_1$–$C_6$-alkoxycarbonyl, $C_3$–$C_4$-alkenoxycarbonyl, $C_1$–$C_6$-alkylthiocarbonyl, or cyano, and
$R^5$ denotes $C_1$–$C_6$-alkyl which is unsubstituted or substituted by lower alkoxy, alkylthio, cyano or by halogen; unsubstituted or halogen-substituted $C_2$–$C_5$-alkenyl; $C_2$–$C_4$-alkynyl; $C_3$–$C_7$-cycloalkyl; a heterocyclic radical which is unsubstituted or substituted by lower alkyl or halogen and which contains one oxygen, one sulfur or one nitrogen atom, or up to two nitrogen atoms and one oxygen atom, or up to nitrogen atoms and sulfur atom; or $R^5$ denotes unsubstituted phenyl or phenyl substituted by from one to three identical or different substituents selected from the group consisting or fluorine, chlorine, bromine, trifluoromethyl, cyano, nitro, $C_1$–$C_4$-alkyl, and $C_1$–$C_4$-alkoxy.

2. A fungicide containing, as one component, a substituted cyanamide as claimed in claim 1.

3. A fungicide as claimed in claim 2, containing a solid or liquid carrier and a substituted cyan amide as claimed in claim 1.

4. Use of a substituted cyanamide as claimed in claim 1 for combating fungi or fungus attack.

5. A process for the production of a substituted cyanamide as claimed in claim 1, characterized in that one mole of an N-halomethylanilide of the formula

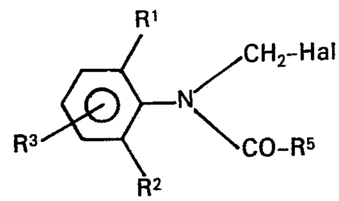

II

where $R^1$, $R^2$, $R^3$ and $R^5$ have the above meanings and Hal denotes halogen, particularly chlorine and bromine, is reacted with at least one mole of a cyanamide of the formula

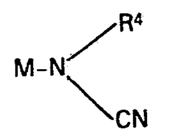

III

where $R^4$ has the above meanings and M denotes hydrogen, an alkali metal or a tetraalkylammonium radical, at from 0° to 150°C, in the presence or absence of an acid binder and in the presence or absence of a solvent inert to the reactants.

6. A process for the production of a substituted cyanamide as claimed in claim 5, characterized in that the reaction is carried out in a two-phase system, the one phase being water and the other a solvent immiscible with the aqueous phase and inert to the reactants, in the presence of from 0.5 to 30 mole% of a phase transfer catalyst.

7. A substituted cyanamide as claimed in claim 1, namely
N-methoxycarbonyl-N-(N'-methoxyacetyl, N'-2',6'-dimethylanilinomethyl)-cyanamide,
N-ethoxycarbonyl-N-(N'-methoxyacetyl, N'-2',6'-dimethylanilinomethyl)-cyanamide,
N-methoxycarbonyl-N-(N'-3-fluorobenzoyl, N'-2',6'-dimethylanilinomethyl)-cyanamide,
N-methoxycarbonyl-N-(N'-4-fluorobenzoyl, N'-2',6'-dimethylanilinomethyl)-cyanamide,
N-methoxycarbonyl-N-(N'-furo-2-yl, N'-2',6'-dimethylanilinomethyl)-cyanamide
N-ethoxycarbonyl-N-(N'-furo-2-yl, N'-2',6'-dimethylanilinomethyl)-cyanamide or
N-methoxycarbonyl-N-(N'-theno-2-yl, N'-2',6'-dimethylanilinomethyl)-cyanamide.

8. A fungicide as claimed in claim 2, containing a substituted cyanamide, namely

N-methoxycarbonyl-N-(N′-methoxyacetyl, N′-2′,6′-dimethylanilinomethyl)-cyanamide,
N-ethoxycarbonyl-N-(N′-methoxyacetyl, N′-2′,6′-dimethylanilinomethyl)-cyanamide,
N-methoxycarbonyl-N-(N′-3-fluorobenzoyl, N′-2′,6′-dimethylanilinomethyl)-cyanamide,
N-methoxycarbonyl-N-(N′-4-fluorobenzoyl, N′-2′,6′-dimethylanilinomethyl)-cyanamide,
N-methoxycarbonyl-N-(N′-furo-2-yl, N′-2′,6′-dimethylanilinomethyl)-cyanamide,
N-ethoxycarbonyl-N-(N′-furo-2-yl, N′-2′,6′-dimethylanilinomethyl)-cyanamide or
N-methoxycarbonyl-N-(N′-theno-2-yl, N′-2′,6′-dimethylanilinomethyl)-cyanamide.

**Claims**

for the Contracting state: AT

1. A fungicide containing, as one component, a substituted cyanamide of the general formula

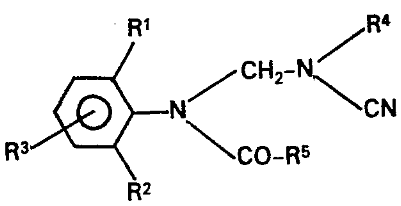

I

where

$R^1$ denotes $C_1$-$C_4$-alkyl or $C_1$-$C_4$-alkoxy,

$R^2$ denotes hydrogen, $C_1$-$C_4$-alkyl, $C_1$-$C_4$-alkoxy or halogen,

$R^3$ denotes hydrogen, $C_1$-$C_4$-alkyl or halogen,

$R^4$ denotes $C_1$-$C_6$-alkoxycarbonyl, $C_3$-$C_4$-alkenoxycarbonyl, $C_1$-$C_6$-alkylthiocarbonyl, or cyano, and

$R^5$ denotes $C_1$-$C_6$-alkyl which is unsubstituted or substituted by lower alkoxy, alkylthio, cyano or by halogen; unsubstituted or halogen-substituted $C_2$-$C_5$-alkenyl; $C_2$-$C_4$-alkynyl; $C_3$-$C_7$-cycloalkyl; a heterocyclic radical which is unsubstituted or substituted by lower alkyl or halogen and which contains one oxygen, one sulfur or one nitrogen atom, or up to two nitrogen atoms and one oxygen atom, or up to two nitrogen atoms and one sulfur atom; or $R^5$ denotes unsubstituted phenyl or phenyl substituted by from one to three identical or different substituents selected from the group consisting of fluorine, chlorine, bromine, trifluoromethyl, cyano, nitro, $C_1$-$C_4$-alkyl, and $C_1$-$C_4$-alkoxy.

2. A fungicide as claimed in claim 1, containing a solid or liquid carrier and a substituted cyanamide as defined in claim 1.

3. Use of a substituted cyanamide as defined in claim 1 for combating fungi or fungus attack.

4. A process for the production of a substituted cyanamide as defined in claim 1, characterized in that one mole of an N-halomethylanilide of the formula

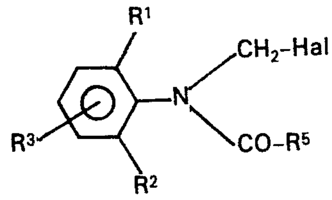

II

where $R^1$, $R^2$, $R^3$ and $R^5$ have the above meanings and Hal denotes halogen, particularly chlorine and bromine, is reacted with at least one mole of a cyanamide of the formula

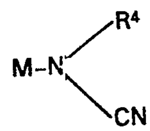

III

where $R^4$ has the above meanings and M denotes hydrogen, an alkali metal or a tetraalkylammonium radical, at from 0° to 150 °C, in the presence or absence of an acid binder and in the presence or absence of a solvent inert to the reactants.

5. A process as claimed in claim 4, characterized in that the reaction is carried out in a two-phase system, the one phase being water and the other a solvent immiscible with the aqueous phase and inert to the reactants, in the presence of from 0.5 to 30 mole% of a phase transfer catalyst.

6. A fungicide as claimed in claim 1, containing a substituted cyanamide, namely
N-methoxycarbonyl-N-(N′-methoxyacetyl, N′-2′,6′-dimethylanilinomethyl)-cyanamide,
N-ethoxycarbonyl-N-(N′-methoxyacetyl, N′-2′,6′-dimethylanilinomethyl)-cyanamide,
N-methoxycarbonyl-N-(N′-3-fluorobenzoyl, N′-2′,6′-dimethylanilinomethyl)-cyanamide,
N-methoxycarbonyl-N-(N′-4-fluorobenzoyl, N′-2′,6′-dimethylanilinomethyl)-cyanamide,
N-methoxycarbonyl-N-(N′-furo-2-yl, N′-2′,6′-dimethylanilinomethyl)-cyanamide,
N-ethoxycarbonyl-N-(N′-furo-2-yl, N′-2′,6′-dimethylanilinomethyl)-cyanamide or
N-methoxycarbonyl-N-(N′-theno-2-yl, N′-2′,6′-dimethylanilinomethyl)-cyanamide.

**Revendications**

pour les Etats contractants BE, CH, DE, FR, GB, IT, LU, NL et SE

1. Cyanamide substitué de formule générale

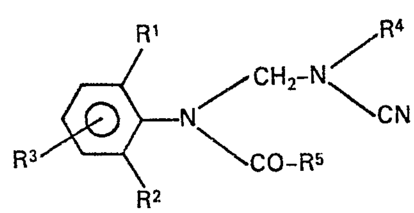

I

dans laquelle

$R^1$ représente un alkyle en $C_1$ à $C_4$ ou un alcoxy en $C_1$ à $C_4$,

$R^2$ l'hydrogène, un alkyle en $C_1$ à $C_4$, un alcoxy en $C_1$ à $C_4$ ou un halogène,

$R^3$ l'hydrogène, un alkyle en $C_1$ à $C_4$ ou un halogène,

$R^4$ un alcoxycarbonyle en $C_1$ à $C_6$, un alcénoxy carbonyle en $C_3$ à $C_4$, un alkyl-thiocarbonyle en $C_1$ à $C_6$ ou le cyanogène, et

$R^5$ un alkyle en $C_1$ à $C_6$, éventuellement substitué par un alcoxy, alkylthio, cyanogène ou halogène, un alcényle en $C_2$ à $C_5$ éventuellement substitué par un halogène, un alcinyle en $C_2$ à $C_4$, un cycloalkyle en $C_3$ à $C_7$, un reste hétérocyclique éventuellement substitué par un alkyle inférieur, ou un halogène, reste qui contient un atome d'oxygène ou un atome de soufre ou un atome d'azote ou jusqu'à deux atomes d'azote et un atome d'oxygène, ou jusqu'à deux atomes d'azote et un atome de soufre,

$R^5$ signifiant en outre un phényle ou un phényle substitué par un à trois substituants identiques ou différents appartenant au groupe fluor, chlore, brome, trifluorométhyle, cyanogène, nitro, alkyle en $C_1$ à $C_4$, alcoxy en $C_1$ à $C_4$.

2. Fongicide contenant, comme un des composants, un cyanamide substitué selon la revendication 1.

3. Fongicide selon la revendication 2, contenant un support solide ou liquide et un cyanamide substitué comme défini à la revendication 1.

4. Utilisation d'un cyanamide substitué comme défini à la revendication 1, pour lutter contre les champignons ou les invasions des champignons.

5. Procédé de préparation d'un cyanamide substitué tel que défini à la revendication 1, caractérisé par le fait qu'on fait réagir à une température de 0 à 150°C éventuellement en présence d'un liant d'acide et éventuellement en présence d'un solvant inerte vis-à-vis des partenaires de réaction, une mole d'un N-halogénométhylanilide de formule

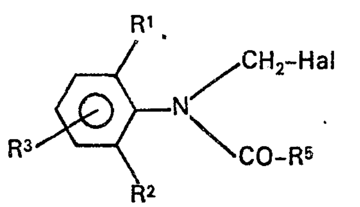

II

dans laquelle $R^1$, $R^2$, $R^3$ et $R^5$ ont les significations indiquées ci-dessus et Hal signifie un halogène, en particulier le chlore ou le brome, avec au moins une mole d'un cyanamide de formule

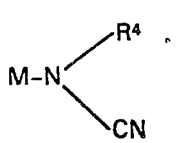

III

dans laquelle $R^4$ a la signification indiquée plus haut et M représente l'hydrogène, un métal alcalin ou un reste tétraalkylammonium.

6. Procédé selon la revendication 5, caractérisé par le fait qu'on effectue la réaction en présence de 0,5 à 30 moles % d'un catalyseur de transfert de phases, dans un système à deux phases, l'une des phases étant l'eau et l'autre phase un solvant inerte vis-à-vis des partenaires de réaction et non miscible avec la phase aqueuse.

7. Cyanamide substitué selon la revendication 1, à savoir

N-méthoxycarbonyl-N-(N'-méthoxyacétyle, N'-2',6'-diméthylanilinométhyl)-cyanamide

N-éthoxycarbonyl-N-(N'-méthoxyacétyle, N'-2',6'-diméthylanilinométhyl)-cyanamide

N-méthoxycarbonyl-N-(N'-3-fluorobenzoyle, N'-2',6'-diméthylanilinométhyl)-cyanamide

N-méthoxycarbonyl-N-(N'-4-fluorobenzoyle, N'-2',6'-diméthylanilinométhyl)-cyanamide

N-méthoxycarbonyl-N-(N'-furo-2-yle, N'-2',6'-diméthylanilinométhyl)-cyanamide

N-éthoxycarbonyl-N-(N'-furo-2-yle, N'-2',6'-diméthylanilinométhyl)-cyanamide

N-méthoxycarbonyl-N-(N'-théno-2-yle, N'-2',6'-diméthylanilinométhyl)-cyanamide.

8. Fongicide selon la revendication 2, contenant un cyanamide substitué, à savoir

N-méthoxycarbonyl-N-(N'-méthoxyacétyle, N'-2',6'-diméthylanilinométhyl)-cyanamide

N-éthoxycarbonyl-N-(N'-méthoxyacétyle, N'-2',6'-diméthylanilinométhyl)-cyanamide

N-méthoxycarbonyl-N-(N'-3-fluorobenzoyle, N'-2',6'-diméthylanilinométhyl)-cyanamide

N-méthoxycarbonyl-N-(N'-4-fluorobenzoyle, N'-2',6'-diméthylanilinométhyl)-cyanamide

N-méthoxycarbonyl-N-(N'-furo-2-yle, N'-2',6'-diméthylanilinométhyl)-cyanamide

N-éthoxycarbonyl-N-(N'-furo-2-yle, N'-2',6'-diméthylanilinométhyl)-cyanamide

N-méthoxycarbonyl-N-(N'-théno-2-yle, N'-2',6'-diméthylanilinométhyl)-cyanamide.

**Revendications**

pour l'Etat contractant: AT

1. Fongicide contenant comme un des composants, un cyanamide substitué de formule générale

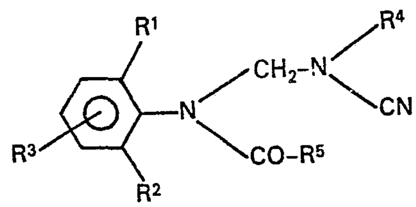

I

dans laquelle

$R^1$ représente un alkyle en $C_1$ à $C_4$ ou un alcoxy en $C_1$ à $C_4$,

$R^2$ l'hydrogène, un alkyle en $C_1$ à $C_4$, un alcoxy en $C_1$ à $C_4$ ou un halogène,

$R^3$ l'hydrogène, un alkyle en $C_1$ à $C_4$ ou un halogène,

$R^4$ un alcoxycarbonyle en $C_1$ à $C_6$, un alcénoxy carbonyle en $C_3$ à $C_4$, un alkyl-thiocarbonyle en $C_1$ à $C_6$ ou le cyanogène, et

$R^5$ un alkyle en $C_1$ à $C_6$, éventuellement substitué

par un alcoxy, alkylthio, cyanogène ou halogène, un alcényle en $C_2$ à $C_5$ éventuellement substitué par un halogène, un alcinyle en $C_2$ à $C_4$, un cycloalkyle en $C_3$ à $C_7$, un reste hétérocyclique éventuellement substitué par un alkyle inférieur, ou un halogène, reste qui contient un atome d'oxygène ou un atome de soufre ou un atome d'azote ou jusqu'à deux atomes d'azote et un atome d'oxygène, ou jusqu'à deux atomes d'azote et un atome de soufre,

$R^5$ signifiant en outre un phényle ou un phényle substitué par un à trois substituants identiques ou différents appartenant au groupe fluor, chlore, brome, trifluorométhyle, cyanogène, nitro, alkyle en $C_1$ à $C_4$, alcoxy en $C_1$ à $C_4$.

2. Fongicide selon la revendication 1, contenant un support solide ou liquide et un cyanamide substitué comme défini à la revendication 1.

3. Utilisation d'un cyanamide substitué comme défini à la revendication 1, pour lutter contre les champignons ou les invasions des champignons.

4. Procédé de préparation d'un cyanamide substitué tel que défini à la revendication 1, caractérisé par le fait qu'on fait réagir à une température de 0 à 150°C éventuellement en présence d'un liant d'acide et éventuellement en présence d'un solvant inerte vis-à-vis des partenaires de réaction, une mole d'un N-halogénométhylanilide de formule

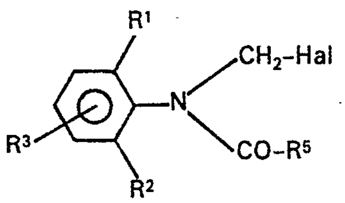

II

dans laquelle $R^1$, $R^2$, $R^3$ et $R^5$ ont les significations indiquées ci-dessus et Hal signifie un halogène, en particulier le chlore ou le brome, avec au moins une mole d'un cyanamide de formule

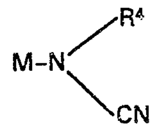

III

dans laquelle $R^4$ a la signification indiquée plus haut et M représente l'hydrogène, un métal alcalin ou un reste tétraalkylammonium.

5. Procédé selon la revendication 4, caractérisé par le fait qu'on effectue la réaction en présence de 0,5 à 30 moles % d'un catalyseur de transfert de phases, dans un système à deux phases, l'une des phases étant l'eau et l'autre phase un solvant inerte vis-à-vis des partenaires de réaction et non nuisible avec la phase aqueuse.

6. Fongicide selon la revendication 1, contenant un cyanamide substitué, à savoir
N-méthoxycarbonyl-N-(N'-méthoxyacétyle, N'-2',6'-diméthylanilinométhyl)-cyanamide
N-éthoxycarbonyl-N-(N'-méthoxyacétyle, N'-2',6'-diméthylanilinométhyl)-cyanamide
N-méthoxycarbonyl-N-(N'-3-fluorobenzoyle, N'-2',6'-diméthylanilinométhyl)-cyanamide
N-méthoxycarbonyl-N-(N'-4-fluorobenzoyle, N'-2',6'-diméthylanilinométhyl)-cyanamide
N-méthoxycarbonyl-N-(N'-furo-2-yle, N'-2',6'-diméthylanilinométhyl)-cyanamide
N-éthoxycarbonyl-N-(N'-furo-2-yle, N'-2',6'-diméthylanilinométhyl)-cyanamide
N-méthoxycarbonyl-N-(N'-théno-2-yle, N'-2',6'-diméthylanilinométhyl)-cyanamide